(19) Europäisches Patentamt
European Patent Office
Office européen des brevets

(11) **EP 2 720 786 B1**

(12) **EUROPEAN PATENT SPECIFICATION**

(45) Date of publication and mention
of the grant of the patent:
**24.01.2018 Bulletin 2018/04**

(21) Application number: **12732805.2**

(22) Date of filing: **15.06.2012**

(51) Int Cl.:
***B01J 8/02*** *(2006.01)*     ***B01J 8/04*** *(2006.01)*

(86) International application number:
**PCT/EP2012/061475**

(87) International publication number:
**WO 2012/172065 (20.12.2012 Gazette 2012/51)**

(54) **METHOD FOR CARRYING OUT EXOTHERMIC CATALYTIC REACTIONS**

VERFAHREN ZUR DURCHFÜHRUNG EXOTHERMER KATALYTISCHER REAKTIONEN

PROCÉDÉ DE RÉALISATION DE RÉACTIONS CATALYTIQUES EXOTHERMIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.06.2011 DK 201100452**

(43) Date of publication of application:
**23.04.2014 Bulletin 2014/17**

(73) Proprietor: **Haldor Topsøe A/S
2800 Kgs. Lyngby (DK)**

(72) Inventor: **THORHAUGE, Max
DK-2730 Herlev (DK)**

(74) Representative: **Haldor Topsøe A/S
Haldor Topsøes Allé 1
2800 Kgs. Lyngby (DK)**

(56) References cited:
**WO-A1-2010/085926**     **US-A- 4 152 407
US-A- 5 190 731**

**Description**

The present invention relates to a method for carrying out exothermic catalytic reactions. Exothermic reactions generate heat and the reactions must be cooled in order to obtain reasonable reaction yields and to prevent destruction of the catalyst and reactor employed for the reactions.

Thus, several catalytic processes have an upper critical temperature limit that may not be exceeded, either due to extensive catalytic deactivation or due to side reactions. However, catalytic reactions have to be performed at elevated or high reaction temperatures in order to obtain appropriate catalytic activity.

The range between the lower reaction temperature and the upper critical temperature will often be smaller than the adiabatic temperature rise, which makes it very difficult to use low cost reactor types like an adiabatic fixed bed reactor or a quench reactor.

The temperature during an exothermic reaction is conventionally controlled by cooling the reaction with a cooling agent in expensive cooled tubular reactors or by dilution of the feed stream in order to reduce the adiabatic temperature rise.

We have found that a possibility to overcome the problem of the adiabatic temperature rise in an adiabatic reactor or the first bed of a quench reactor, is to establish an internal bypass in the fixed catalytic bed, and to provide a heat transfer area in such a way that the reaction heat from the catalyst section is transferred to the internal bypass section. As an example, if half amount of feed gas is by-passed the catalyst bed, and the reaction heat of the catalyst section is transferred to both the by-passed stream and the reacting stream, the temperature rises to half of the adiabatic temperature rise.

Since there is no pressure difference between the catalyst section and the bypass section, the heat transfer area can be made of thin plate steel at low cost.

Because of the reactor must be able to operate both at high and low load, it is, however, important that the ratio of bypass flow and the flow through the catalyst bed is almost constant. This can be achieved when the pressure drop dependency on the flow rate in the catalyst bed is identical to that in the bypass section. The pressure drop over a catalyst bed typically depends on the square of the flow. In order to keep a constant bypass/ catalyst flow ratio the bypass section must obey the same dependency.

We have found that that a constant bypass/ catalyst flow ratio and a by-pass flow dependency on the square of the flow can be provided if the pressure drop in the bypass section is subjected to momentum loss or loss of velocity head, i.e. the velocity of a fluid expressed in terms of the head or static pressure required to produce that velocity.

Pursuant to the above findings and observations, this invention provides a method for performing an exothermic catalytic reaction comprising the steps of

providing a feed gas stream comprising reactants for the exothermic catalytic reaction to a fixed bed catalytic reactor comprising one or more catalyst beds each with catalyst particles filled sections with a catalyst volume (VCAT) ;

providing a feed gas bypass inside the reactor by arranging within at least one of the catalyst beds a number of bypass passageways without catalytic active particles inside the passageways having a cooling surface area (ACOOL);

passing a part of the feed gas stream through the bypass passageways and reminder of the stream through the catalyst particles filled sections;

removing heat from the reacting feed gas stream being passed through the catalyst filled sections by an indirect heat transfer to the part of the feed gas stream being passed through the bypass passageways;

adjusting the catalyst volume (VCAT) and the cooling surface (ACOOL) so that the ratio of the catalyst volume to the cooling surface (VCAT/ACOOL) is between 0.008m and 0.08m;

and adjusting the total superficial area (Ab)of the bypass passageways, so that ratio of the total superficial area (Ab) of the bypass passageways to the total superficial area (Ac)of the catalyst filled sections results in a value of M between 0.7 and 1.3,

where

$$Ab/Ac = M * ((dTad - dT)/ dT) * ((Kb/Kc)^{(0.5)});$$

Kb is the friction loss coefficient of the bypass passageways given as the number of velocity heads

$$Kb = dPf / (0.5*DENS*(U^2)),$$

Kc is the friction loss coefficient of the catalyst filled section given as the number of velocity heads

$$Kc = dPf / (0.5*DENS*(U^2));$$

and

dTad [°C] is the potential adiabatic temperature rise of the feed gas if the exothermic reaction proceeds to equilibrium under adiabatic conditions,
dT [°C] is a predetermined acceptable temperature increase in the catalytic reactor,
DENS [kg/m3] is the density of the feed gas,
U [m/s] is the superficial gas velocity of the feed gas, and dPf [Pa] is the frictional pressure drop.

In a specific embodiment of the invention, the ratio of the catalyst volume (VCAT) to the cooling surface (ACOOL) is between 0.01m and 0.04m, and the total superficial area (Ab) of the bypass passageways is adjusted to provide a value of M between 0.9 and 1.2.

In still a specific embodiment of the invention, the feed gas stream is passed in series through at least two catalyst beds provided with the bypass passageways.

In further a specific embodiment of the invention, the feed gas stream being withdrawn form the at least one catalyst bed with the bypass passageways is further passed through a fixed catalyst bed without bypass passageways and being operated in adiabatic manner.

In further a specific embodiment of the invention, the feed gas stream being withdrawn from the at least one catalyst bed is cooled by heat exchange or by quench cooling with a feed gas stream.

The method can be carried out in a catalytic reactor for performing exothermic reactions in a feed gas stream comprising within a common shell
one or more catalyst beds each with catalyst particles filled sections and a catalyst volume (VCAT);
a number of bypass passageways without catalyst particles arranged within at least one of the catalyst beds, the by passageways having a cooling surface area (ACOOL), wherein the ratio of the catalyst volume (VCAT) to the cooling surface area (ACOOL) is between 0.008m and 0.08m;
the ratio of the total superficial area (Ab) of the bypass passageways to the total superficial area (Ac) of the catalyst filled sections has a value M of between 0.7 and 1.3, where

$$Ab/Ac = M * ((dTad - dT)/ dT) * ((Kb/Kc)^(0.5));$$

Kb is the friction loss coefficient of the bypass passageways given as the number of velocity heads

$$Kb = dPf / (0.5*DENS*(U^2)),$$

Kc is the friction loss coefficient of the catalyst filled section given as the number of velocity heads

$$Kc = dPf / (0.5*DENS*(U^2));$$

and

dTad [°C] is the (feasible) potential adiabatic temperature rise of the feed gas if the exothermic reaction proceeds to equilibrium under adiabatic conditions,
dT [°C] is a predetermined acceptable (set) temperature increase in the catalytic reactor,
DENS [kg/m3] is the density of the feed gas,
U [m/s] is the superficial gas velocity of the feed gas, and
dPf [Pa] is the frictional pressure drop.

In a specific embodiment of the above catalytic reactors the ratio of the catalyst volume (VCAT) to the cooling surface (ACOOL) is between 0.01m and 0.04m, and the total superficial area of the bypass passageways (Ab) is has a value of M between 0.9 and 1.2.

In further a specific embodiment the catalytic reactor comprises at least two catalyst beds arranged in series and each provided with the bypass passageways.

In still a specific embodiment the catalytic reactor further comprises an adiabatic catalyst bed without the bypass passageways.

In another specific embodiment the catalytic reactor further comprises an adiabatic operating catalyst bed.

In a further specific embodiment the catalytic reactor comprises one or more heat exchanger arranged between the catalyst beds or inlet means for a cool feed gas stream between the catalyst beds.

There are several means to achieve pressure drop dependency of the flow rate in the catalyst bed being identical to that in the bypass passageways.

In accordance with a specific embodiment the bypass passageways are filled with catalytic inactive particles.

In another embodiment nozzle or perforated plates are arranged within the bypass passageways.

In further an embodiment the bypass passageways are formed of metallic sheets arranged in parallel and spaced apart and/or of tubes.

In further an embodiment the above metallic sheets and/or tubes forming the bypass passageways contain cross-corrugated structured elements or the metallic plates are in corrugated shape.

The main advantage of the above described method according to the invention is that they allow use of a cheap rector type in processes where the adiabatic temperature rise is too high. The invention provides additionally reduced recycle dilution for control of the adiabatic temperature rise.

The invention will be described in further detail in the following with reference to the accompanying drawings in which

Fig. 1a to 1c show different shapes of a bypass passage way for use in the invention;

Fig. 2 is a cross-section view of a catalyst bed provided with bypass passageways according to a specific embodiment of the invention; and

Fig. 3 shows a cross-section view of a bypass passage way provided with cross corrugated element according to a specific embodiment of the invention.

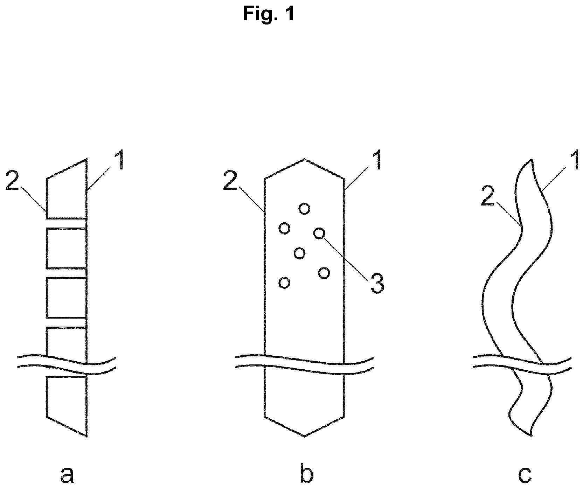

Referring to Fig.1, the bypass passageway of Fig. 1a consists of a straight rear wall 1 and a folded front wall 2. The bypass passage way of Fig. 1b consist of two walls 1 and 2 in parallel and spaced apart. The space between the plates may be filled with catalytic inert particles 3.

The bypass passageway of Fig. 1c consists of two corrugated plates 1 and 2. In all the shown embodiments, the end portions of the plates are assembled in order to create a closed space between the plates.

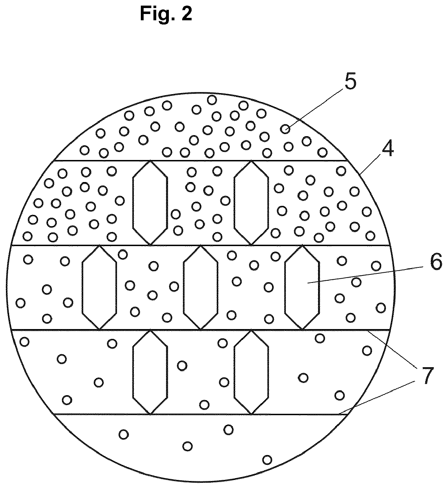

Fig. 2 is a cross section through a catalytic bed 4 filled with catalyst particles 5. Bypass passageways 6, for instance in the shape shown in Fig. 1b are inserted into the catalyst bed and surrounded by the catalyst particles. The bypass passageways are suspended between support beams 7.

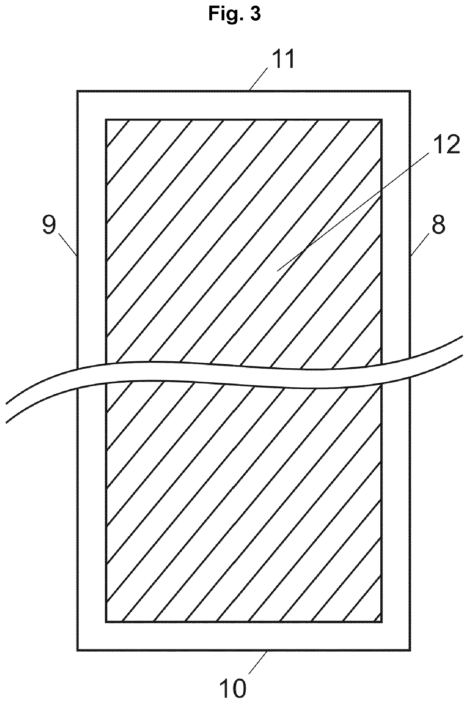

Fig. 3 shows a bypass passageway with a rectangular cross sectional shape formed by plates 8, 9, 10 and 11 provided with a cross-corrugated element 12 inside the passageway. The outer walls of element 12 are spaced apart from inner walls of plates 8, 9, 10 and 11.

Example 1

Reactor design and process conditions for a method and reactor of the above discussed type are determined by means of the following equations based on predetermined values as explained below.

For the catalytic conversion of a feed gas containing 90 mol% methanol vapour ($CH_3OH(g)$) and 10 mol% water ($H_2O(g)$) to dimethyl ether ($CH_3OCH_3(g)$) and water ($H_2O(g)$) at 1.5MPa reactor pressure, it is preferred to have an inlet temperature of 340°C in order to have a high catalyst activity, and a temperature below 410°C in order to avoid side reactions forming hydrocarbons. If the reaction proceeds to equilibrium under adiabatic conditions the temperature rise will be 101°C (dTad), resulting in an exit temperature of 441°C out of the catalyst bed.

In order to reduce the catalyst temperature and to avoid side reactions, the above method according to the invention is used by means of the following predetermined values:

A catalyst filled section in form of 2m high reactor filled with 5mm catalyst pellets has a friction loss coefficient of the catalyst filled section (catalyst bed) Kc of 22,716.

In order to provide bypass passageways within the reactor for cooling the catalyst, we employ 2m long channels formed of metal plates with a corrugation height of 4.5mm and a corrugation angle of 60° resulting in friction loss coefficient of the bypass passageways Kb of 202 (Fig.1c, Fig.2 and Fig.3). Each passageway is 0.4m wide and has a superficial area (Ab) of 0.0018m$^2$ and an effective cooling area (ACOOL) of 1.6m$^2$.

The temperature rise is reduced to 60°C (dT) by adjusting the ratio of the total superficial area (Ab) of the bypass passageways to the total superficial area (Ac) of the catalyst filled section to 0.0677, calculated according to the above defined formula: Ab/Ac= 1.05 * ((101°C - 60°C)/ 60°C )* ((202/22716)^0.5) by choosing a value of M= 1.05. Since each bypass passageway has a superficial area of 0.0018m$^2$, 38 channels (0.0677m2/0.0018m2/channel) must be installed for each superficial square meter of the catalyst filled section.

Since each bypass passageway has a cooling area of 1.6m$^2$, the ratio VCAT/ACOOL = (2m * 1m2)/(38 * 1.6m2) = 0.033m. This ratio is within the predefined range. If the ratio VCAT/ACOOL is larger than 0.033m, the friction loss (pressure drop) of the bypass channel is too low, and a lower corrugation height must be used. If the ratio VCAT/ACOOL is lower than 0.033m, the friction loss (pressure drop) of the bypass channel is too high and a larger corrugation height must be used.

Downstream the bypassed cooled catalyst bed, the gas is cooled by addition of 160°C hot unconverted feed gas in order to obtain a temperature of the mixture of 340°C. This mixture is converted over an adiabatic catalyst bed without bypass cooling. Since a large part of the gas already is converted in the bypass cooled catalyst bed, the temperature rise of the adiabatic bed will only be 60°C.

Example 2

Reactor design and process conditions for a method and reactor according to the invention are determined by means of the following equations based on the below predetermined values.

For the catalytic conversion of a given feed gas containing methanol vapour (CH3OH(g)) to gasoline at 2MPa, the methanol content of the gas corresponds to an adiabatic temperature rise of 160°C (dTad). It is preferred to have an inlet temperature of 340°C in order to obtain a high catalyst activity, and a temperature below 420°C in order to avoid fast catalyst deactivation. If the reaction proceeds without cooling the exit temperature out of the catalyst bed will be 340°C + 160°C = 500°C.

In order to reduce the catalyst temperature rise and to avoid side reactions, the reactor design and the method according to the invention are used with the following predetermined values and parameters.

A catalyst filled section in form of 4m high reactor filled with 2mm catalyst pellets has a friction loss coefficient of the catalyst filled section (catalyst bed) Kc of 85,185.

To provide bypass passageways and to cool the catalyst, 4m bypass passageways sections are installed filled with 5mm pellets without catalytic activity having a friction loss coefficient Kb of 37,074. The bypass passageways are separated from the catalyst section by metal plates in order to prevent mixing of the gasses between the catalyst bed and the bypass passageways, but at the same time allow indirect heat transfer.

The temperature rise shall be reduced to 80°C (dT) by adjusting the ratio Ab/Ac= 1.00 * ((160°C - 80°C)/ 80°C)* ((34074/85185)^0.5)=0.632 by choosing M=1.0. The superficial area of the bypass passageways must then be 0.632m$^2$ for each superficial square meter of the catalyst filled section.

Using a predetermined value of VCAT/ACOOL=0.02m, the width (Wc) of the catalyst section, identical to the spacing between the bypass passageways is calculated. Since each catalyst section is adjacent to two bypass passageways VCAT/ACOOL= Wc/2 or the width of the catalyst section is 0.04m. Since Ab/Ac is given to be 0.632, the width of the bypass section (Wb) must be Wb= 0.04m * 0.632 = 0.025m.

Downstream the bypassed cooled catalyst bed the gas is cooled by indirect heat exchange to 340°C followed by a final conversion over an adiabatic catalyst bed without cooling. Since half of the gas was converted in the bypass cooled catalyst bed, the temperature rise of the adiabatic bed is reduced to 80°C.

**Claims**

1. A method for performing an exothermic catalytic reaction comprising the steps of
   providing a feed gas stream comprising reactants for the exothermic catalytic reaction to a fixed bed catalytic reactor comprising one or more catalyst beds (4) each with catalyst particles filled sections with a catalyst volume (VCAT);
   providing a feed gas bypass inside the reactor by arranging within at least one of the catalyst beds a number of bypass passageways (6) without catalytic active particles inside the passageways having a cooling surface area (ACOOL); passing a part of the feed gas stream through the bypass passageways and remainder of the stream through the catalyst particles filled sections;
   removing heat from the reacting feed gas stream being passed through the catalyst filled sections by indirect heat transfer to the part of the feed gas stream being passed through the bypass passageways (6);
   adjusting the catalyst volume (VCAT) and the cooling surface (ACOOL) so that the ratio of the catalyst volume to the cooling surface (VCAT/ACOOL) is between 0.008m and 0.08m;
   and adjusting the total superficial area (Ab) of the bypass passageways (6), so that ratio of the total superficial area (Ab)of the bypass passageways (6) to the total superficial area (Ac)of the catalyst filled sections results in a value of M between 0.7 and 1.3, where

$$Ab/Ac = M * ((dTad - dT)/ dT) * ((Kb/Kc)^(0.5));$$

Kb is the friction loss coefficient of the bypass passageways given as the number of velocity heads

$$Kb = dPf / (0.5*DENS*(U^2)),$$

Kc is the friction loss coefficient of the catalyst filled section given as the number of velocity heads

$$Kc = dPf / (0.5*DENS*(U^2));$$

and

dTad [°C] is the potential adiabatic temperature rise of the feed gas if the exothermic reaction proceeds to equilibrium under adiabatic conditions,
dT [°C] is a predetermined acceptable temperature increase in the catalytic reactor,
DENS [kg/m3] is the density of the feed gas,
U [m/s] is the superficial gas velocity of the feed gas, and
dPf [Pa] is the frictional pressure drop.

2. The method of claim 1, wherein the ratio of the catalyst volume to the cooling surface VCAT/ACOOL is between 0.01m and 0.04m, and wherein the total superficial area (Ab)of the bypass passageways (6) is adjusted to result in a value M of between 0.9 and 1.2.

3. The method according to claim 1 or 2, wherein the feed gas stream is passed in series through at least two catalyst beds (4) provided with the bypass passageways.

4. The method according to anyone of claims 1 to 3, wherein the feed gas stream being withdrawn from the at least one catalyst bed is cooled by heat exchange or by quench cooling with a feed gas stream prior to further conversion.


**Patentansprüche**

1. Verfahren zur Durchführung einer exothermen katalytischen Reaktion, umfassend die Schritte:

Zuführen eines Speisegasstroms enthaltend Reaktanden für die exotherme katalytische Reaktion in einen katalytischen Festbett-Reaktor, umfassend ein oder mehrere Katalysatorbetten (4), jeweils mit Katalysatorpartikeln gefüllten Abschnitten mit einem Katalysatorvolumen (VCAT);
Bereitstellen eines Speisegas-Bypasses im Inneren des Reaktors durch Anordnen einer Anzahl von Bypass-Leitungen (6) ohne katalytisch aktive Partikel innerhalb der Leitungen in mindestens einem der Katalysatorbetten, die eine kühlende Oberfläche (ACOOL) aufweisen;
Leiten eines Teils des Speisegasstroms durch die Bypass-Leitungen und der Restmenge des Stroms durch die mit Katalysatorpartikeln gefüllten Abschnitte;
Entfernen von Wärme aus dem reagierenden Speisegasstrom, der durch die mit Katalysator gefüllten Abschnitte geleitet wird, durch indirekte Wärmeübertragung auf den Teil des Speisegasstroms, der durch die Bypass-Leitungen (6) geleitet wird;
Einstellen des Katalysatorvolumens (VCAT) und der Kühlfläche (ACOOL), so dass das Verhältnis des Katalysatorvolumens zur Kühlfläche (VCAT / ACOOL) zwischen 0,008 m und 0,08 m liegt; und
Einstellen der gesamten Flächenausdehnung (Ab) der Bypass-Leitungen, so dass das Verhältnis der gesamten Flächenausdehnung (Ab) der Bypass-Leitungen (6) zur gesamten Flächenausdehnung (Ac) der mit Katalysator gefüllten Abschnitte zu einem Wert von M zwischen 0,7 und 1,3 führt,

wobei

$$Ab / Ac = M * ( (dTad - dT) / dT) * ( (Kb / Kc) {}^{\wedge} (0,5) );$$

Kb der Reibungsverlustkoeffizient der Bypass-Leitungen ausgedrückt als Wert des dynamischen Drucks ist

$$Kb = dPf / (0,5 * DENS * (U^{\wedge}2) ),$$

Kc der Reibungsverlustkoeffizient der Bypass-Leitungen ausgedrückt als Wert des dynamischen Drucks ist

$$Kc = dPf / (0,5 * DENS * (U {}^{\wedge} 2) );$$

und

dTad [°C] der potentielle adiabatische Temperaturanstieg des Speisegases ist, wenn die exotherme Reaktion unter adiabatischen Bedingungen zum Gleichgewichtszustand fortschreitet,
dT [°C] eine vorbestimmte akzeptable Temperaturerhöhung im katalytischen Reaktor ist,
DENS [kg / m$^3$] die Dichte des Speisegases ist,
U [m / s] die Oberflächengasgeschwindigkeit des Speisegases ist und
dPf [Pa] der Reibungsdruckabfall ist.

2. Verfahren gemäß Anspruch 1, wobei das Verhältnis des Katalysatorvolumens zu der Kühloberfläche VCAT / ACOOL zwischen 0,01 m und 0,04 m liegt und wobei die gesamte Flächenausdehnung (Ab) der Bypass-Leitungen (6) so eingestellt wird, dass sie zu einem Wert M zwischen 0,9 und 1,2 führt.

3. Verfahren gemäß Anspruch 1 oder 2, wobei der Speisegasstrom in Reihe durch mindestens zwei Katalysatorbetten (4) geführt wird, die mit den Bypass-Leitungen versehen sind.

4. Verfahren gemäß einem der Ansprüche 1 bis 3, wobei der Speisegasstrom, der von dem mindestens einen Katalysatorbett abgezogen wird, vor der weiteren Umwandlung durch Wärmeaustausch oder durch Abschreckungskühlen mit einem Speisegasstrom gekühlt wird.

**Revendications**

1. Procédé destiné à réaliser une réaction catalytique exothermique comprenant les étapes
fournir un courant de gaz d'alimentation comprenant des réactifs pour la réaction catalytique exothermique à un réacteur catalytique à lit fixe comprenant un ou plusieurs lits de catalyseur (4), chacun avec des sections remplies de particules de catalyseur avec un volume de catalyseur (VCAT);
fournir une dérivation de gaz d'alimentation à l'intérieur du réacteur en agençant au sein d'au moins un des lits de catalyseur un certain nombre de passages de dérivation (6) sans particules actives catalytiques à l'intérieur des passages ayant une superficie de refroidissement (ACOOL) ;
faire passer une partie du courant de gaz d'alimentation à travers les passages de dérivation et le reste du courant à travers les sections remplies de particules de catalyseur ;
enlever la chaleur du courant de gaz d'alimentation en cours de réaction qui est passé à travers les sections remplies de catalyseur par un transfert de chaleur indirect jusqu'à la partie du courant de gaz d'alimentation qui est passée à travers les passages de dérivation (6) ;
ajuster le volume de catalyseur (VCAT) et la surface de refroidissement (ACOOL) de sorte que le rapport entre le volume de catalyseur et la surface de refroidissement (VCAT/ACOOL) se situe entre 0,008 m et 0,08 m ;
et ajuster la surface spécifique totale (Ab) des passages de dérivation (6), de sorte que le rapport entre la surface spécifique totale (Ab) des passages de dérivation (6) et la surface spécifique totale (Ac) des sections remplies de catalyseur se traduit par une valeur de M entre 0,7 et 1,3,
où

$$Ab/Ac = M * ((dTad - dT)/dT) * ((Kb/Kc) \char94 (0,5)) ;$$

Kb est le coefficient de perte de frottement des passages de dérivation donné en tant que nombre de charges dynamiques

$$Kb = dPf / (0,5 * DENS * (U \char94 2)),$$

Kc est le coefficient de perte de frottement des sections remplies de catalyseur donné en tant que nombre de charges dynamiques

$$Kc = dPf / (0,5 * DENS * (U \char94 2)) ;$$

et

dTad [°C] est la montrée en température adiabatique potentielle du gaz d'alimentation si la réaction exothermique continue jusqu'à l'équilibre dans des conditions adiabatiques,
dT [°C] est une augmentation de température acceptable prédéterminée dans le réacteur catalytique,
DENS [kg/m3] est la densité du gaz d'alimentation,
U [m/s] est la vélocité de gaz superficielle du gaz d'alimentation, et
dPf [Pa] est la chute de pression par frottement.

2.  Procédé selon la revendication 1, dans lequel le rapport entre le volume de catalyseur et la surface de refroidissement VCAT/ACOOL se situe entre 0,01 et 0,04 m, et dans lequel la surface spécifique totale (Ab) des passages de dérivation (6) est ajustée pour se traduire par une valeur de M entre 0,9 et 1,2.

3.  Procédé selon la revendication 1 ou 2, dans lequel le courant de gaz d'alimentation est passé en série à travers au moins deux lits de catalyseur (4) équipés des passages de dérivation.

4.  Procédé selon l'une quelconque des revendications 1 à 3, dans lequel le courant de gaz d'alimentation qui est retiré de l'au moins un lit de catalyseur est refroidi par échange de chaleur ou par refroidissement par extinction avec un courant de gaz d'alimentation avant de poursuivre la conversion.

**Fig. 1**

a                b                c

**Fig. 2**

**Fig. 3**